(19) Europäisches Patentamt

European Patent Office

Office européen des brevets

(11) **EP 2 172 239 A2**

(12) **EUROPEAN PATENT APPLICATION**

(43) Date of publication:
**07.04.2010 Bulletin 2010/14**

(51) Int Cl.:
***A61M 15/00*** *(2006.01)*

(21) Application number: **09172206.6**

(22) Date of filing: **05.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **06.10.2008 JP 2008259233**

(71) Applicant: **Canon Kabushiki Kaisha Tokyo 146-8501 (JP)**

(72) Inventors:
• **Fukumoto, Yoshiyuki**
  **Tokyo 146-8501 (JP)**
• **Kobayashi, Masaya**
  **Tokyo 146-8501 (JP)**
• **Sugita, Masaru**
  **Tokyo 146-8501 (JP)**

(74) Representative: **TBK-Patent**
  **Bavariaring 4-6**
  **80336 München (DE)**

(54) **Discharge head and droplet discharging device**

(57)    A discharge head 10 for discharging droplets from discharge ports can withstand high water pressure and is provided with a first orifice plate 3 having first discharge ports 3a and a second orifice plate 6 having second discharge ports. The first orifice plate 3 and the second orifice plate 6 are separated from each other in the liquid discharge direction of the discharge head 10 and disposed opposite to each other. The diameter of the discharge ports of the second orifice plate 6 is smaller than the diameter of the discharge ports 3a of the first orifice plate 3 so that very fine droplets 9 are discharged from the discharge head 10 as the liquid discharged from the discharge ports 3a of the first orifice plate 3 is split by the second discharge ports.

FIG. 1

EP 2 172 239 A2

**Description**

BACKGROUND OF THE INVENTION

Field of the Invention

The present invention relates to a discharge head for discharging liquid such as liquid medicine and turning the liquid into liquid droplets and also to a droplet discharging device.

Related Background Art

A method of administering a drug to a patient by using an inhaler and causing the patient to inhale very fine droplets of the drug (liquid medicine) formed by dispersing the drug into a solution is known. Such an inhaler includes a reservoir for storing liquid medicine, a discharge head for discharging the liquid medicine and a control section for controlling the operation of each of the components. Particularly, for a patient to inhale liquid medicine by way of alveoli of the lung, droplets should be made to represent a diameter not greater than 10 $\mu$m so as to be able to get to alveoli. Therefore, the inhaler is required to be able to produce a large number of droplets having such a small droplet diameter.
Many discharge methods have been proposed to date and inhalers using such methods have been developed and put to practical use. Popular discharging methods include one for vibrating liquid medicine by means of ultrasonic waves and producing droplets from the surface of the liquid medicine and atomizing them by vibrations in order to allow the patient to inhale the drug. A technique of applying a voltage to a piezoelectric element to oscillate the element and generate ultrasonic waves has been and is being popularly employed. A filtering method of allowing only small droplets to pass through a mesh that is a thin film having a plurality of very small holes in order to improve the distribution of diameters of atomized droplets has also been proposed.
Other known discharge methods include one for arranging a heater/heating mechanism near the discharge ports of an inhaler, energizing the heater to boil liquid and discharging droplets from the discharge ports (see International Publication WO 95/01137). A discharge head for using this method includes a heater board base member where a liquid-chamber/flow-path forming member and heater wiring are arranged and an orifice plate that is a member having discharge ports and bonded to the base member. While this method allows the inhaler to be downsized and droplets to be micronized with ease, it is accompanied by problems including that large power needs to be supplied to the heater to increase the discharge rate so that it is actually difficult to increase the discharge rate and that the drug can be scorched by heat. Liquid medicine may be discharged from discharge ports by applying pressure to the liquid medicine by way of displacement of piezoelectric crystals so as to push out droplets from the discharge ports as disclosed in WO 95/01137.
Still other known discharge methods include one for guiding liquid medicine under high pressure to the discharge ports of a discharge head and spraying droplets from the discharge ports at high speed (pressure exertion method). With such a method, the liquid medicine passes through fine discharge ports and a liquid jet that is a continuous fast flow of liquid is discharged from each of the discharge ports. Particularly, when the diameter of the discharge ports is small, the liquid jets are crushed to droplets by the wave that is autonomously generated along the lateral surface of the liquid jets to consequently produce droplets when the liquid jets proceed from the respective discharge ports by a certain distance. The distance from a discharge port that is required for a liquid jet to be crushed is referred to as droplet forming length. The droplet forming length can vary depending on the velocity of the liquid jet, the diameter of the discharge ports, the surface tension of the liquid medicine and the viscosity of the liquid medicine. The liquid medicine is discharged as liquid jets from the discharge ports until the liquid jets proceed by the droplet forming length but then discharged as droplets beyond the droplet forming length. A pressure exertion method provides advantages including that it is free from the problem of scorching the liquid medicine that accompanies the method of WO 95/01137, that the power required to discharge the liquid medicine can be reduced and that a high discharge rate can be achieved with ease.
Meanwhile, with a pressure exertion method, the meniscus pressure that is produced at the discharge ports due to the surface tension of the liquid medicine increases as the diameter of the discharge ports is reduced to consequently raise the discharge pressure necessary for discharging the liquid medicine as liquid jets. Since a liquid jet is produced when the inertial force of the liquid jet surpasses the surface tension, the condition to be met for a liquid jet to be discharged can be determined by the Weber number defined by formula (1) depicted below:

$$\rho \times D \times V^2 / \sigma, \qquad (1)$$

where $\rho$ is the liquid density, D is the diameter of the discharge port, V is the velocity of the liquid jet at the discharge port surface and $\sigma$ is the surface tension of the liquid. A liquid jet is discharged when the value of the formula (1) exceeds a certain level. The velocity of the liquid jet at this time is defined as liquid jet forming velocity $V_d$. If the discharge pressure in the discharge head that corresponds to the liquid jet forming velocity $V_d$ is $P_d$, $P_d$ has a term that is proportional to the square of $V_d$ and, approximately, since a liquid jet is discharged with a constant value of the formula (1), the discharge pressure increases as the nozzle diameter is reduced.

Droplets of a size in the order of several microns need to be produced for a discharge head to be effectively used in an inhaler. When droplets of a size in the order of several microns are discharged from a discharge head with a pressure exertion method, the discharge pressure will be not less than 2 MPa as described in International Publication WO 94/27653. Therefore, inhalers adopting a pressure exertion method are required to have a structure that can withstand high liquid pressure at the part thereof that is brought into contact with liquid medicine and also a mechanism for exerting high pressure so that they face a problem of durability and that of downsizing. In another technical field, or the field of printers, printers of a continuous ink-jet type adopt a pressure exertion method and employ a pump as pressure exerting mechanism so that printers are inevitably large.

WO 94/27653 discloses an inhaler adopting such a pressure exertion method. According to the patent document, a discharge head and a reservoir are combined to form an integral structure, which is a cartridge, and the material of the cartridge depicts plasticity. When administering a drug, a piston is pushed out by spring force to partly crush the cartridge and produce large pressure that discharges liquid medicine from the discharge ports. With this method, the cartridge is replaced each time after administering a drug. The cartridge is disposable and this method is referred to as a single dose type, whereas a method of administering a drug for several times without replacing the cartridge or the head is referred to as a multi-dose type.

A single dose inhaler as disclosed in WO 94/27653 requires the cartridge to be replaced each time after drug administration to make the operation of the inhaler cumbersome and liable to induce an accident. Additionally, the cost of the cartridge material rises. Therefore, it is desirable to realize a multi-dose inhaler using a pressure exertion method. For this purpose, the discharge head is required to represent a sufficient degree of durability and withstand high liquid pressure after a plurality of times of drug administration. Additionally, the inhaler needs to be downsized from the viewpoint of portability. For this purpose, the inhaler requires low discharge pressure and also a simplified structure for each part of the inhaler.

The discharge pressure of an inhaler is the sum of the pressure energy necessary for discharging droplets at the discharge velocity $V_d$ or at a higher velocity and the pressure loss $P_L$ at the discharge port. The $P_L$ at an ordinary discharge port can approximately be expressed by formula (2) depicted below that conforms to the Hagen-Poiseuille's equation:

$$P_L = (C \times \mu \times T \times V) / D^2, \qquad (2)$$

where $\mu$ is the viscosity of droplets, T is the thickness of the orifice plate and C is a coefficient of proportion that is determined according to the structure of the orifice plate. From the formulas (1) and (2), it will be seen that the discharge pressure can be reduced by modifying the physical properties of the liquid medicine and the design of the orifice plate. The surface tension and the viscosity of the liquid medicine cannot be modified remarkably from the viewpoint of maximizing the effect of the drug. As for the orifice plate, the value of $P_L$ of the formula (2) is reduced by increasing the diameter of the discharge ports, increasing the number of discharge ports and/or reducing the thickness of the orifice plate to enable to reduce the discharge pressure.

Generally, the diameter and the number of the discharge ports cannot normally be modified because they determine the droplet diameter and the discharge rate respectively. On the other hand, the pressure loss can be reduced at the discharge ports to lower the discharge pressure by reducing the thickness of the orifice plate. However, the strength of the orifice plate is reduced when the orifice plate is made thin to make it difficult to bond the orifice plate and the discharge head. Even if they can be bonded, the orifice plate can no longer withstand the high liquid pressure at the time of discharging the liquid medicine to give rise to liquid leakage from the junction and destroy the discharge head. Additionally, the orifice plate itself will be deformed and destroyed under the high liquid pressure.

It is difficult for the conventional design of arranging an orifice plate at part of the container for containing solution under high water pressure as disclosed in WO 94/27653 to secure durability because the orifice plate is exposed to high water pressure for a long time when the discharge head is operated repeatedly. However, durability does not give rise to a serious problem when the orifice plate is exposed to high water pressure only instantaneously in instances where the discharge head is disposable as disclosed in WO 94/27653.

Additionally, the problem that the durability of the discharge head is reduced as the orifice plate is made thinner to reduce the discharge pressure remains unsolved.

SUMMARY OF THE INVENTION

In view of the above-identified circumstances, it is therefore the object of the present invention to provide a discharge head and a droplet discharging device that can produce very small droplets than ever while maintaining the durability of the discharge head without raising the pressure necessary for discharging liquid.

In an aspect of the present invention, the above object is achieved by providing a discharge head to be used for a droplet discharging device for discharging liquid from discharge ports by exerting pressure, the discharge head including a first orifice plate having first discharge ports of a first discharge port diameter, a second orifice plate arranged at a position separated from the first orifice plate in the liquid discharge direction and having second discharge ports of a second discharge port diameter, the second discharge port diameter being smaller than the first discharge port diameter.

The arrangement of two orifice plates provides the advantage as described below. As the second orifice plate of the second discharge port diameter smaller than the first discharge port diameter is provided, the first discharge port diameter of the first orifice plate can be made larger than the diameter of the droplets to be produced. Then, as a result, it is not necessary to raise the discharge pressure. Thus, it is no longer necessary to make the first orifice plate thin and hence the discharge head can maintain a high degree of durability.

Other features and advantages of the present invention will be apparent from the following description taken in conjunction with the accompanying drawings, in which like reference characters designate the same or similar parts throughout the figures thereof.

BRIEF DESCRIPTION OF THE DRAWINGS

FIG. 1 is a schematic cross-sectional view of a discharge head of the first embodiment of the present invention, illustrating the state thereof in a liquid discharging operation.

FIG. 2 is a schematic cross-sectional view of the discharge head of FIG. 1, illustrating the state thereof when the discharge head is not discharging liquid.

FIG. 3 is a schematic cross-sectional view of a discharge head obtained by modifying the first embodiment.

FIG. 4 is a schematic cross-sectional view of an inhaler formed by using a discharge head as illustrated in FIG. 1.

FIG. 5 is a schematic cross-sectional view of a discharge head according to the second embodiment of the present invention.

FIG. 6 is a schematic cross-sectional view of a known discharge head.

FIG. 7 is a graph illustrating the relationship between the discharge port diameter and the discharge pressure of each sample of discharge head in an experiment for comparison.

DESCRIPTION OF THE PREFERRED EMBODIMENTS

Preferred embodiments of the present invention will now be described in detail in accordance with the accompanying drawings.

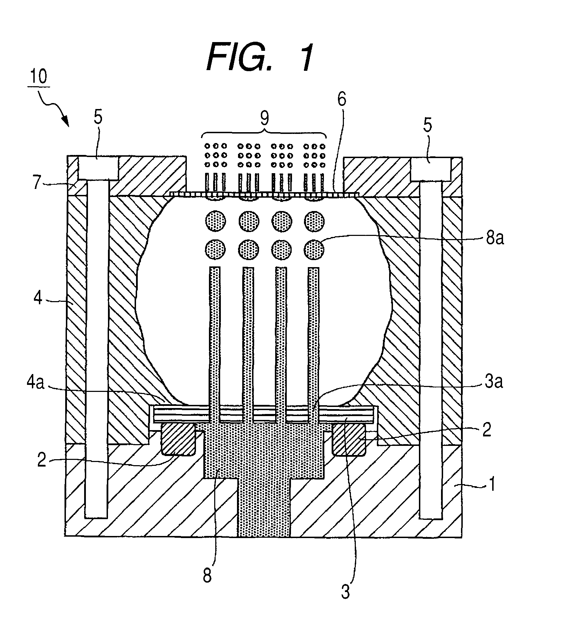

FIG. 1 illustrates a discharge head 10 of the first embodiment of the present invention. The head base member 1 of the discharge head 10 that is mounted in an inhaler as a droplet discharging device supports a seal member 2 and a first orifice plate 3. Of a first plate securing member 4 that is a wall member, the bottom part 4a holds an outer peripheral part of the first orifice plate 3 and pushes the first orifice plate 3 and the seal member 2 against the head base member 1. The pressure necessary for the push is generated as screws 5 are driven to run through the first plate securing member 4 and engage with the respective screw holes formed in the head base member 1. A separate wall member may be provided apart from the first plate securing member 4 that operates to secure the first orifice plate 3 and also as a wall member.

A second orifice plate 6 is arranged opposite to and separated in the liquid discharge direction from the first orifice plate 3 and rigidly held in position by a second plate securing member 7. The first plate securing member 4 and the second plate securing member 7 are rigidly secured on the head base member 1 in the above mentioned order in order to control the position of the second orifice plate 6. Holes are cut through the second plate securing member 7 so as not to block the liquid flow paths and the second orifice plate 6 is made to bond the second plate securing member 7 by way of a peripheral part thereof.

The first orifice plate 3 has first discharge ports 3a. The second orifice plate 6 has second discharge ports (not illustrated) having a diameter smaller than the first discharge ports 3a. The flows of the liquid 8 discharged from the first discharge ports 3a become droplets 8a and collide with the second orifice plate 6 and the flows of the liquid 8 are split further by the second discharge ports so that very fine droplets 9 having a designed droplet diameter are produced when the initial droplets pass through the second discharge ports.

Preferably, the liquid jets discharged from the first discharge ports 3a can be turned into droplets by the time they get to the second orifice plate 6 and then forced to collide with the second discharge ports as droplets as illustrated in FIG. 1. This is because liquid can pass through the second discharge ports more easily when the liquid is forced to collide with the first discharge ports as droplets rather than as liquid jets. The reason for this is that the droplets 8a in FIG. 1 are produced as the liquid jets are crushed to represent a diameter that is twice as large as the diameter of the liquid jets so that the quantity of the liquid that strikes a unit area of the second orifice plate 6 is reduced and liquid can pass through the second discharge ports more easily. As the droplets 8a collide with the second discharge ports, the loss of the liquid medicine (liquid) that cannot pass through the second discharge ports can be reduced to a large extent. Therefore, it is desirable to make the distance (gap) separating the second orifice plate 6 and the first orifice plate 3 not smaller than the droplet forming length.

The droplet forming length is small when the droplet diameter is small. When the first discharge ports have a diameter of not less than 10 $\mu$m, liquid jets can advantageously be turned into droplets when the distance between the second orifice plate 6 and the first orifice plate 3 is not less than 300 $\mu$m.

If the second orifice plate 6 is separated from the first orifice plate 3 too much, on the other hand, the velocity of the liquid jets discharged from the first discharge ports 3a falls under the influence of the resistance of air, the gravity, the air flow in the discharge head and so on and the moving directions of the liquid jets can be disturbed. It has been found that the problem is insignificant when the distance separating the two orifice plates is not more than 50 mm. From the above, the distance between the second orifice plate 6 and the first orifice plate 3 is preferably not less than 50 $\mu$m and not more than 50 mm.

A plurality of first discharge ports 3a may be provided or, alternatively, a single discharge port 3a may be provided. The discharge rate rises as the number of discharge ports increases. First discharge ports 3a are formed by the number necessary for achieving the desired discharge rate. When a plurality of first discharge ports 3a are provided, the distance separating adjacent discharge ports is so selected as to avoid allowing the droplets discharged from the first discharge ports 3a to collide with each other. The distance can be at least not less than the discharge port diameter (first discharge port diameter) of the first discharge ports 3a.

Referring to FIG. 1, high positive pressure is exerted to the liquid 8 by a pressure exerting mechanism (not illustrated) that is a mechanism for exerting discharge pressure at the side of the liquid supply port of the discharge head 10. A high degree of durability is required to the head base member 1, the seal members 2 and the first orifice plate 3 because they are exposed to high water pressure. Therefore, it is necessary for the first orifice plate 3 to have a thickness that prevents the first orifice plate 3 from being destroyed under water pressure of the liquid 8. It is also desirable that the first orifice plate 3 is not deformed under water pressure. Once the first orifice plate 3 is deformed, the discharged liquid can no longer strike the surface of the second orifice plate 6 perpendicularly. Then, as a result, the liquid faces difficulty in passing through the second orifice plate 6. The thickness of the first orifice plate 3 is within a range between 20 $\mu$m and 5 mm for realizing a strong orifice plate. The first orifice plate 3 is free from any risk of being deformed when the first orifice plate 3 is made thick and strong. Then, the first orifice plate 3 can be directly pushed against the seal member 2 and rigidly held there.

Preferably, the first discharge ports 3a have a diameter that is large to a certain extent. This is because the pressure loss can increase at the discharge ports when the orifice plate 3 is made thick as evidenced by the formula (2). Since the pressure loss is inversely proportional to the square of the diameter of the discharge ports as evidenced by the formula (2), the increase of the pressure loss can be suppressed sufficiently by raising the diameter of the discharge ports even if the orifice plate 3 is made thick. However, the droplet forming length of the liquid discharged from the first discharge ports 3a can rise to make the liquid collide with the second orifice plate as droplets if the diameter of the discharge ports is increased carelessly.

Therefore, the diameter of the first discharge ports 3a is preferably not less than 10 $\mu$m and not more than 500 $\mu$m.

The second discharge ports cut through the second orifice plate 6 are made to represent a diameter (of the second discharge ports) that can discharge droplets with an intended droplet diameter. In other words, the diameter of the second discharge ports is made smaller than the diameter of the first discharge ports 3a. The number of discharge ports of the second orifice plate 6 is preferably greater than the number of discharge ports of the first orifice plate 3 in order to efficiently turn each of the droplets discharged from the first discharge ports 3a with a relatively large diameter into a plurality of droplets of a smaller diameter. Since it is difficult to accurately align the second discharge ports with the first discharge ports 3a, the second discharge ports are preferably arranged regularly and highly densely like so many meshes. No problem arises if the first discharge ports 3a and the second discharge ports are not aligned accurately provided that a plurality of second discharge ports are formed over a large region of the second orifice plate 6.

The diameter of the second discharge ports is preferably within a range between 0.1 and 10 $\mu$m, more preferably between 3 $\mu$m and 7 $\mu$m, because such a range is suitable for an inhaler by means of which a user inhales liquid medicine. The diameter of the second discharge ports may be appropriately selected for an application other than an inhaler.

When the distance between adjacent second discharge ports is large, the droplets discharged from the first discharge ports 3a can collide with only a small number of second discharge ports so that droplets can pass through the second orifice plate 6 only with difficulty. At worst, only a small part of the droplets discharged from the first discharge ports 3a passes through the second discharge ports while most of the droplets remain on the rear surface of the second orifice plate 6 to produce a large loss on the part of the liquid medicine. When the distance between adjacent second discharge ports is increased further to become greater than the diameter of the first discharge ports, some of the droplets discharged from the first discharge ports 3a may not collide with any of the second discharge ports. Therefore, the distance between adjacent second discharge ports needs to be not more than the diameter of the first discharge ports. Conversely, when the distance between adjacent second discharge ports is decreased to become smaller than the diameter of the second discharge ports, many of the droplets discharged form the second discharge ports may become united. Therefore, the distance between adjacent second discharge ports needs to be not less than the diameter of the second discharge ports.

The second orifice plate 6 is made thin. At least the second orifice plate 6 is made thinner than the first orifice plate 3. This is to reduce the pressure loss defined by the formula (2). Since no hydrostatic pressure is exerted to the second orifice plate 6, the thickness of the second orifice plate 6 can be reduced to a minimum level without taking its strength into consideration. Preferably, the thickness of the second orifice plate 6 is within a range between 20 and 0.1 $\mu$m.

A suitable profile of the orifice plates of a discharge head according to the present invention will be described below from the viewpoint of making a high durability and a reduced pressure loss compatible with each other. Assume here that the pressure difference between the internal pressure of a discharge head adopting the pressure exertion method as illustrated in FIG. 1 that is raised by a pressure exerting mechanism and the atmospheric pressure is $P_0$.

Since $P_0$ is approximately divided into a pressure loss and the kinetic energy of liquid jets according to the Bernoulli's theorem, formula (3) depicted below holds true.

$$P_0 = P_L + \frac{\rho V^2}{2} = \frac{C\mu TV}{D^2} + \frac{\rho V^2}{2} \qquad \ldots (3)$$

From the formula (3) above, the formula (2) for pressure loss can be rewritten to formula (4) depicted below.
$$P_L = \left(\frac{C\mu T}{D^2}\right)^2 \frac{1}{\rho}\left[\sqrt{1+\left(\frac{D^2}{C\mu T}\right)^2 2\rho P_0} - 1\right] \qquad \ldots (4)$$

Assume that the diameter of the discharge ports and the thickness of the first orifice plate 3 are respectively $D_1$ and $T_1$, while the diameter of the discharge ports and the thickness of the second orifice plate 6 are respectively $D_2$ and $T_2$. Additionally, assume that $\rho$, C and $\mu$ take respective values that are substantially common to all the orifice plates for the purpose of simplification. Since the first orifice plate 3 is required to represent a high strength, $T_1$ needs to represent a certain large value. $D_2$ needs to represent a value good for the target droplet diameter. When compared with the pressure loss that arises when liquid is discharged only by means of a first orifice plate having a thickness of $T_1$ and a discharge port diameter $D_2$ as in the prior art, the relationship of formula (5) depicted below is required to a discharge head according to the present invention in order to reduce the pressure loss that arises when liquid is discharged with the discharge port diameter $D_2$ of the second orifice plate 6.
$$\left(\frac{T_1}{D_2{}^2}\right)^2\left[\sqrt{1+\left(\frac{D_2{}^2}{C\mu T_1}\right)^2 2\rho P_0}-1\right] > \left(\frac{T_1}{D_1{}^2}\right)^2\left[\sqrt{1+\left(\frac{D_1{}^2}{C\mu T_1}\right)^2 2\rho P_0}-1\right]$$

$$(5)$$

$$+\left(\frac{T_2}{D_2{}^2}\right)^2\left[\sqrt{1+\left(\frac{D_2{}^2}{C\mu T_2}\right)^2 2\rho P_0\left(1-\frac{P'}{P_0}\right)}-1\right]$$

The left side of the formula (5) is the pressure loss of a discharge head of the prior art, whereas the right side is the pressure loss of a discharge head according to the present invention. In the formula (5), P' is the first term of the right side. The formula (5) can be simplified to formula (6) depicted below when an ordinary solution and orifice plates having an ordinary profile are employed and $P_0$ is not extremely small.
$$\frac{T_1}{D_2{}^2} > \frac{T_1}{D_1{}^2}+\frac{T_2}{D_2{}^2}\sqrt{1-\frac{P'}{P_0}} > \frac{T_1}{D_1{}^2}+\frac{T_2}{D_2{}^2} \qquad ..(6)$$

As pointed out above, the first orifice plate 3 and the second orifice plate 6 are designed to satisfy the requirements depicted below.
$$T_1 > T_2 \qquad \ldots (7)$$

$$D_1 > D_2 \qquad \ldots (8)$$

Thus, the orifice plates 3 and 6 that satisfy the requirements of the formulas (6), (7) and (8) should be designed for a discharge head according to the present invention in order to reduce the pressure loss relative to any prior art discharge heads, while maintaining a necessary degree of strength for the orifice plates.
Note that the formula (6) can be modified to be "$(T_1 - T_2) / D_2{}^2 > T_1 / D_1{}^2$".
The first orifice plate 3 is required to be rigid and strong and hence the first orifice plate 3 is thick and has large discharge ports. In other words, the first orifice plate 3 can be prepared with ease and made of any of a variety of materials. Suitable materials of the first orifice plate 3 include inorganic oxides, inorganic nitrides and inorganic carbides. Specific examples of such materials include oxides, nitrides and carbides of aluminum, silicon, titanium, tantalum, zirconium, hafnium, niobium, magnesium, iron, manganese and chromium. The material of the first orifice plate 3 may contain a plurality of elements selected from those listed above. Alternatively, the material of the first orifice plate 3 may be made of ordinary stainless steel, aluminum, nickel, palladium, iron, titanium, silicon, manganese, chromium, tantalum or a compound of any of them. Still alternatively, the material of the first orifice plate 3 may be made of a polymeric organic resin such as polycarbonate, epoxy, vinyl chloride, paraxylylene or polyimide.
The second orifice plate 6 needs to be thin and able to be formed with fine discharge ports. Additionally, the second orifice plate 6 is desirably water repellent both at the front surface and at the rear surface. Then, the second orifice plate 6 can prevent from being wetted and encourage liquid medicine to be discharged from fine holes while the liquid medicine can be collected from the discharge ports as will be described hereinafter. Generally speaking, when a plate is made water repellent, its adhesiveness is reduced. However, a water repellent second orifice plate 6 does not give rise to any problem because the second orifice plate 6 is required neither to be adhesive nor to be water-pressure-proof. Materials that can suitably be used for the second orifice plate include compounds containing fluorine. Other suitable materials include polymeric organic resins such as polycarbonate, epoxy, vinyl chloride, paraxylylene and polyimide. Alternatively, the materials may be metals such as aluminum, nickel, palladium, iron, titanium, silicon, manganese, chromium, tantalum or a combination of them. It is desirable that the second orifice plate is coated with a thin film prepared by using a compound containing fluorine when it is made of a material other than a compound containing fluorine.

The contact angle of liquid relative to the surface of the second orifice plate 6 is preferably greater than the contact angle of liquid relative to the surface of the first orifice plate 3. This is because the first orifice plate 3 is held in contact with the liquid medicine in the head and preferably not so strongly water repellent from the viewpoint of smoothly feeding the liquid medicine to the discharge ports, whereas the second orifice plate 6 has small discharge ports and it is necessary to make the second orifice plate 6 easily discharge liquid by raising its water repellency. Additionally, a small contact angle of the first orifice plate 3 is suitable from the viewpoint of collecting liquid at the side of the first orifice plate 3 with ease.

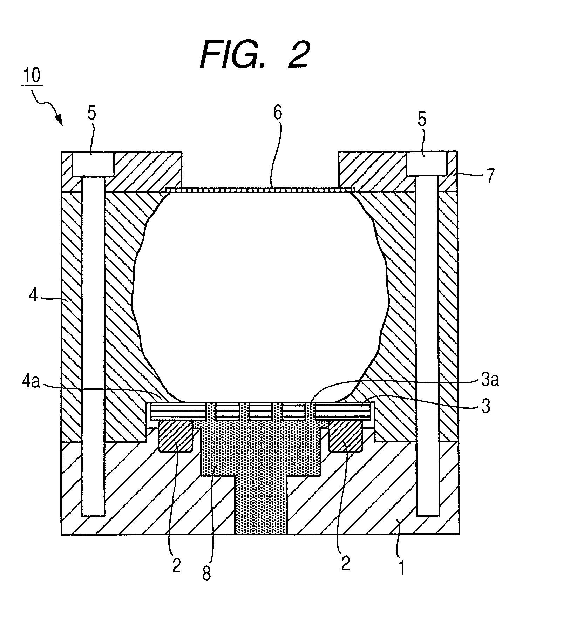

FIG. 2 is a schematic cross-sectional view of the discharge head 10 of FIG. 1, illustrating the state thereof when the discharge head is in storage. To end the discharge from the discharge head 10, the positive internal pressure in the head is reduced to nil by the pressure exerting mechanism. Preferably, the internal pressure is lowered and held to a negative pressure level within a range between 1 and 20 kPa. Liquid can be collected from the discharge ports and any leakage of liquid medicine can be prevented by holding the internal pressure to a negative pressure level. The liquid in the head can be held stably by the surface tension of the meniscuses existing in the first discharge ports 3a.

The liquid that cannot pass through the second orifice plate 6 constitutes a loss of liquid medicine. However, the liquid medicine can be consumed without being wasted when the liquid is collected in the head. With a technique of collecting such liquid, the bottom part 4a of the plate securing member 4 found between the first orifice plate 3 and the second orifice plate 6 is made to represent a conical profile. Then, as a result, the liquid medicine adhering to the inner surface of the second orifice plate 6 and that of the first orifice plate 4 can be collected to the first discharge ports 3a by gravity. The surface of the first plate securing member 4 and that of the second orifice plate 6 are advantageously highly water repellent. The liquid medicine collected to and near the first discharge ports 3a can be collected to the inside of the discharge head from the first discharge ports 3a by the negative pressure exerted thereto when the discharge head is in storage. Preferably, the inner wall of the plate securing member 4 represents a curved profile and the surface of the inner wall is smooth. Preferably, the cross section of the hollow space defined by the plate securing member 4 that is taken along a plane running in parallel with the surfaces of the orifice plates becomes smaller as the plane approaches the first orifice plate 3 and the second orifice plate 6. When the cross section of the hollow space defined by the plate securing member 4 is minimized at the top and at the bottom in this way, the liquid medicine that cannot pass through the second orifice plate 6 can be forced to move smoothly to the first orifice plate 3. The liquid medicine can be driven to move smoothly by arranging a mechanism for blowing air and/or a wiper mechanism for wiping the inner wall surface in addition to utilizing the gravity.

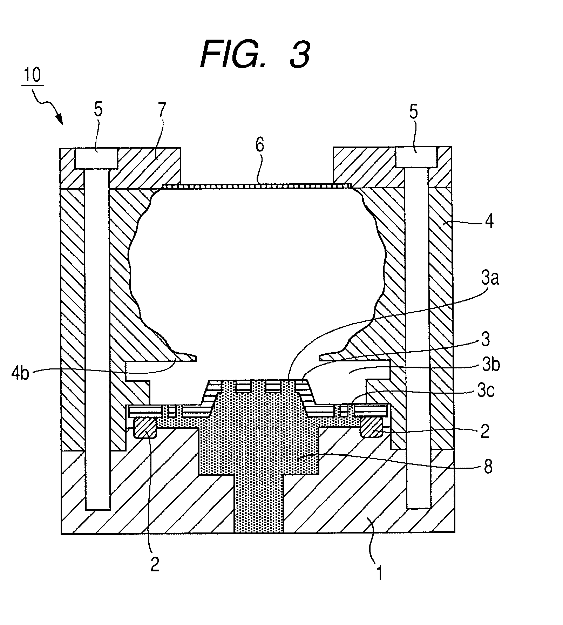

FIG. 3 is a schematic cross-sectional view of a discharge head obtained by modifying the first embodiment. This modified embodiment is provided with a separate liquid collection unit and liquid is not intentionally collected from the first discharge ports 3a. The orifice plate 3 is worked in such a way that the part of the orifice plate 3 where the discharge ports 3a are formed projects in the discharging direction so that the liquid to be collected does not accumulate in the discharge ports 3a when it is driven to move toward the orifice plate 3. The orifice plate 3 is provided with a liquid pool section 3b, which operates to collect liquid, and liquid collection ports 3c at a part thereof that does not project. Liquid collection ports may be arranged in the head base member 1 or the plate securing member 4.

The structure of the discharge head including the profile of the plate securing member 4 is optimized so as to collect liquid to and near the liquid collection ports 3c. When pressure is exerted to the liquid 8 and liquid jets are discharged from the first discharge ports 3a in a discharging operation, liquid can be discharged from the liquid collection ports 3c at the same time. Therefore, eaves 4b are arranged in front of the liquid collection ports 3c so that the liquid discharged from the liquid collection ports 3c may collide with the eaves 4b, then move along the plate securing member 4 and return to the liquid collection ports 3c again so as to be collected before getting to the second orifice plate 6. Since it is not desirable that liquid is discharged from the liquid collection ports 30, the diameter of the liquid collection ports 3c is preferably made smaller than the diameter of the discharge ports 3a in order not to allow the liquid collection port to discharge liquid with ease. The thickness of the liquid collection ports 3c can be made thicker than the thickness of the first discharge ports 3a. A liquid pool section 3b is arranged in front of the liquid collection ports 3c to allow liquid to accumulate there. Then, liquid is hardly discharged from the liquid collection ports 3c because of the provision of the liquid pool section 3b and the liquid from the second orifice plate 6 automatically accumulate in the liquid pool section 3b. Additionally, liquid can easily be discharged from the first discharge ports 3a because no liquid accumulates in front of them.

It is not necessary to collect the discharged liquid that does not pass through the second discharge ports in applications where liquid medicine (liquid) may be lost to a certain extent. In such a case, the plate securing member 4 only takes a role of rigidly holding the second orifice plate 6 in position and hence it is not necessary to completely cover the space between the first orifice plate 3 and the second orifice plate 6. For example, the plate securing member 4 may be one or more pillars.

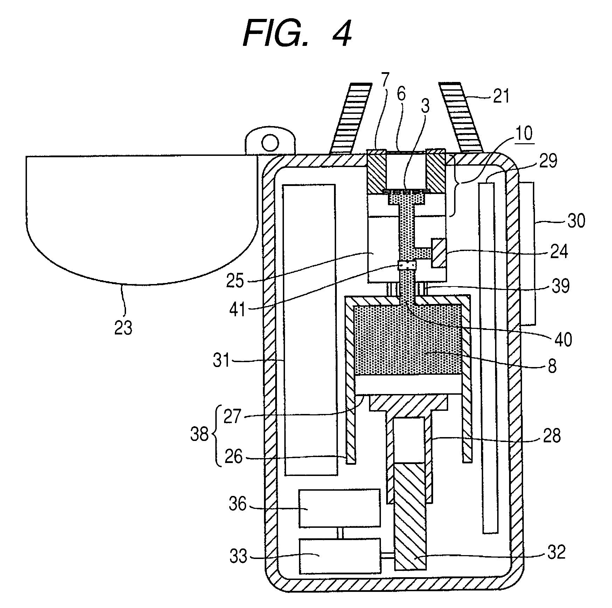

FIG. 4 is a schematic cross-sectional view of an inhaler formed by using a discharge head 10 according to the present invention. Referring to FIG. 4, a reservoir cartridge 38 is formed by a cylinder 26 that operates as a container containing liquid 8, which is liquid medicine, and a closure member 27 that can move, sliding in the cylinder 26. The communication port 40 at the front end of the cylinder 26 communicates with a cartridge connection section 39 at the main body side so that the liquid medicine in the cylinder 26 is transferred up to the discharge head 10. The reservoir cartridge 38 and the main body can be separated from each other. A valve may be provided between the reservoir cartridge 38 and the discharge head 10 to interrupt the communication between them. The reservoir cartridge 38 will be replaced when the cartridge 38 becomes empty. A filter 41 and a pressure sensor 24 are arranged between the reservoir cartridge 38 and the discharge head 10. The filter 41 is preferably a mesh like member so as to operate to capture the dust penetrating into the discharge head 10. The filter 41 may alternatively be arranged in the discharge head 10. The pressure sensor 24 monitors the pressure of the entire liquid medicine in the communication channel.

A pressure exerting mechanism is provided as mechanism for exerting discharge pressure to the liquid medicine in the discharge head 10. More specifically, a droplet discharging device according to the present invention supplies pressurized liquid to the discharge head and causes the discharge head to discharge droplets from the discharge ports of the discharge head. The pressure exerting mechanism includes a piston 28 that is connected to the closure member 27 of the reservoir cartridge 38 and reciprocates in the cylinder 26, a shaft 32 put into the hole of the piston 28, a gear box 33 and a motor 36, the gear box 33 and the motor 36 being adapted to drive the shaft 32 to rotate. The shaft 32 and the piston 28 are helically threaded at parts thereof that contact each other and engaged with each other. The piston 28 reciprocates as the shaft 32 is driven to rotate in opposite directions. Thus, the pressure being applied to the liquid medicine is increased and decreased as the piston 28 and the closure member 27 are driven to reciprocate by the rotating motor 36.

An inhalant pipe 21 is provided and the patient (user) inhales the discharged liquid medicine by way of the inhalant pipe 21. The inhalant pipe 21 is so designed as to externally cover the discharge head 10 and retain the discharged droplets in the pipe 21. The patient brings the inhalant pipe 21 close to his or her nose or mouth and inhales the droplets to take the drug that is turned into droplets into the body. As the patient ends the use of the inhaler, he or she puts the cap 23 on the inhalant pipe 21 for storage.

The inhaler is electrically driven by a control circuit section 29 and a battery 31. The control circuit section 29 drives the motor 36 to rotate and push the piston 28 so as to exert positive pressure to the liquid medicine and cause droplets to be discharge from the discharge head 10, observing the pressure being exerted to the liquid medicine by way of the pressure sensor 24. As the liquid medicine is discharged for a predetermined time period, the control circuit section 29 quickly draws back the piston 28 to put the liquid medicine under negative pressure and end the discharge operation. The liquid medicine in the inhaler can be stored stably by maintaining the negative pressure to a right level. The quantity of the discharged drug is computed from the discharge pressure and the discharge time and the inhaled quantity of drug is stored in the memory of the control circuit section 29 as history. Thus, the patient can constantly grasp the administered and inhaled quantity of drug each day so that a right quantity of drug may be administered and inhaled each time.

When all the liquid medicine in the cylinder 26 of the inhaler of FIG. 4 is gone and the cylinder 26 becomes empty, the piston 28 and the closure member 27 are disconnected and only the reservoir cartridge 38 is replaced. Thus, the inhaler has a multi-dose feature and can be used to administer a drug for a very large number of times. While the discharge head 10 is exposed to very high positive pressure when administering a drug, the discharge head 10 of the present invention is very durable and hence can be operated stably and repeatedly. While the second orifice plate 6 has small discharge ports and the latter can be clogged with ease depending on the drug to be administered, the second orifice plate 6 can be replaced with ease because the reservoir cartridge 38 and the second orifice plate 6 do not communicate with each other. Alternatively, the second orifice plate 6 may be displaced slightly to use the discharge ports thereof with which the droplets discharged from the first orifice plate 3 have not collided. The discharge ports of the first orifice plate 3 have a large diameter and hence coagulation of drug and/or a similar problem hardly takes place at the discharge ports. When the discharge head 10 is broken, the head 10 may be detached from a liquid medicine flow path member 25 and replaced. Flow paths are formed in the liquid medicine flow path member 25 to supply pressurized liquid to the discharge head 10.

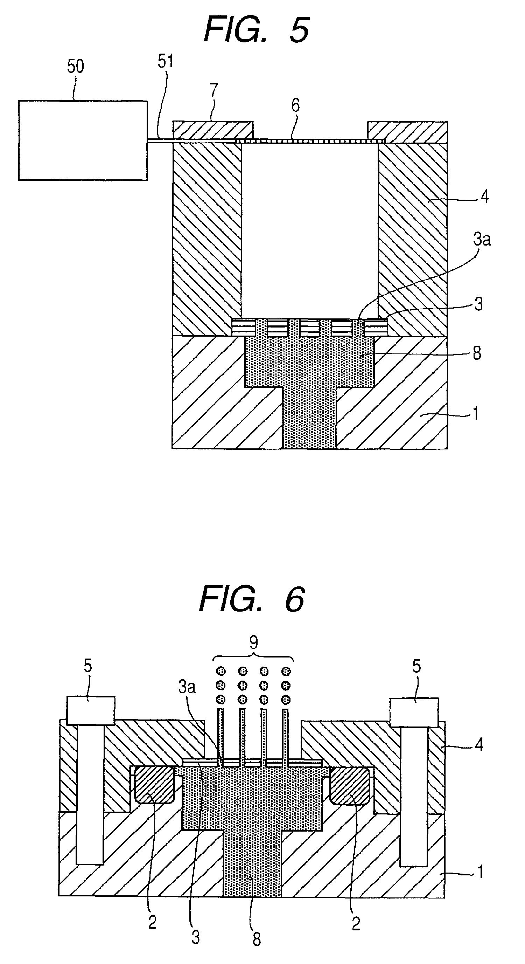

FIG. 5 is a schematic cross-sectional view of a discharge head of the second embodiment of the present invention. While the second embodiment has substantially the same configuration as the first embodiment, it differs from the first embodiment in that the energy generated by an energy application mechanism 50 is applied to part or all of the second orifice plate 6 through an energy transfer member 51. While energy can take any of a variety of forms, thermal energy can be listed above all. When applying thermal energy to the second orifice plate 6, an electric heater may be used for the energy application mechanism 50. The surface tension of the droplets passing through the second orifice plate 6 is lowered as the second orifice plate 6 is connected to the electric heater by wiring and heated. Then, the discharge velocity is also lowered as seen from the formula (1) so that the discharge pressure is also lowered. Additionally, droplets would not easily adhere to the orifice plate 6 when the latter is heated so that the droplets can pass through the second orifice plate 6 smoothly. Thus, a problem that the second discharge ports become wet and prevent droplets from being discharged can be avoided. Since the second orifice plate 6 needs to be heated only for a droplet discharge operation, which is a very short operation, it is desirable that the second orifice plate 6 is heated and cooled quickly. Additionally, it is desirable that the heater heats only the second orifice plate 6 locally. For these reasons, the second orifice plate 6 is preferably made of metal such as a Ni alloy.

Energy in another form is oscillation energy. The second orifice plate 6 may be oscillated in the discharge direction or in a direction perpendicular to the discharge direction. As the second orifice plate 6 is oscillated, kinetic energy is applied to the droplets passing through the second orifice plate 6 to lower the discharge pressure. Additionally, droplets are split further to pass through the second discharge ports with ease. An oscillator such as a piezoelectric body may be used for the energy application mechanism 50 and linked to the second orifice plate 6 by means of an oscillation member to oscillate the second orifice plate 6 in a desired direction. Preferably, the second orifice plate 6 is not rigidly held to the plate securing member 4 so that the second orifice plate 6 can be driven for micro-oscillations. The frequency of oscillation is preferably from 1 KHz to 10 MHz from the viewpoint of quickly applying energy to droplets.

Energy in still another form is charging energy. A voltage application mechanism such as a booster circuit may be used for the energy application mechanism 50. As a preferable example, the second orifice plate 6 is electrically floating and the booster circuit can apply a high voltage to the second orifice plate by way of wiring. The droplets discharged from the first orifice plate 3 are electrically charged and accelerated by the voltage of the second orifice plate 6. The droplets acquire larger kinetic energy as a result of the acceleration of the droplets so that they can pass through the second orifice plate 6 more easily to allow the discharge pressure to be lowered. Alternatively, an electrode for applying a high voltage may be arranged separately near the second orifice plate instead of using the second orifice plate 6 as electrode for applying a high voltage. The electrode and the members surrounding the electrode are preferably coated with an insulator to avoid electric leak. A charging electrode may be arranged between the first orifice plate 3 and the second orifice plate 6 to electrically charge the drug, or the droplets thereof to be accurate, more reliably.

This embodiment can be realized by moving the second orifice plate for discharging very fine droplets away from the first orifice plate that is exposed to high water pressure. In the case of prior art discharge heads, the orifice plates are exposed to high water pressure and deformed so that it is difficult to apply energy by heating or charging. The energy application mechanism may not necessarily be formed by a single kind of energy application mechanism. In other words, the energy application mechanism may be formed by combining two or more different kinds of energy application mechanism.

While the above-described embodiments are inhalers having a discharge head for discharging liquid medicine, the liquid to be discharged by a discharge head according to the present invention is not limited to liquid medicine and a discharge head according to the present invention can be used as general purpose discharge head. Liquid that can be discharged from a discharge head according to the present invention includes liquid medicines, refined water, aromatic solutions, ethanol, ink, functional organic solutions and functional metal solutions. A droplet discharging device according to the present invention can find applications other than inhalers such as humidifiers, aroma generators, printers, mist generators and apparatus for manufacturing electronic devices (displays, wiring boards, etc.). A droplet discharging device using a discharge head according to the present invention is advantageous when compared with prior art droplet discharging devices from the viewpoint of improving the discharge durability and reducing the discharge pressure. It is remarkably and particularly advantageous for discharging very fine droplets.

Examples

A discharge head having a configuration as illustrated in FIG. 1 was prepared and compared with prior art discharge heads.

For the purpose of reference, prior art discharge heads will be described below by referring to FIG. 6.

A seal member 2 and a plate securing member 4 are embedded into a head base member 1. The plate securing member 4 pushes the seal member 2 against the head base member 1 by means of screws 5. As the seal member 2 is deformed under pressure, the seal member 2 hermetically seals the gap between the plate securing member 4 and the head base member 1 to prevent liquid 8 from leaking to the outside. The seal member 2 is preferably a resilient member such as an O-ring or a rubber packing. The plate securing member 4 is provided with a hole so as not to block the flow paths. An outer peripheral part of orifice plate 3 and the plate securing member 4 are bonded to each other typically by means of an adhesive agent. The orifice plate 3 is rigidly held to the plate securing member 4. The orifice plate 3 is provided with discharge ports 3a and constantly exposed to the liquid 8 in the inside of the head so that the plate 3 is frequently subjected to high water pressure. High positive pressure is applied to the liquid 8 by a pressure exerting mechanism (not illustrated) arranged at the liquid feeding side. When the positive pressure exceeds the discharge pressure of the discharge head, liquid jets are discharged from the discharge ports 3a to produce droplets. The diameter of the discharge ports is so defined as to be able to discharge intended droplets 9. The diameter of the discharge ports that are suitable for an inhaler is within a range between 0.1 and 10 μm. To make such a small diameter feasible for the discharge ports, the orifice plate 3 needs to be thin in order to reduce the pressure loss defined by the above formula (2). For this reason, a thickness of not more than 20 μm is selected for the orifice plate 3 more often than not.

Even if the pressure loss is reduced sufficiently, the pressure that needs to counter the surface tension at the discharge ports 3a is still high and the discharge pressure will be almost 1 MPa. Then, as a result, such a thin orifice plate almost always becomes deformed. If the orifice plate 3 is made thinner in order to reduce the pressure loss, it is difficult to make the orifice plate 3 successfully bond the plate securing member 4 so that the orifice plate 3 becomes more easily deformable and crushable. In short, it is necessary to exert high pressure in order to produce desired very fine droplets by means of a one-stage discharge head and then the orifice plate needs to be made very thin in order to reduce the pressure loss that inevitably arises with such an arrangement. Then, the net result will be a poor durability of the discharge head.

Prior art discharge heads having a configuration as illustrated in FIG. 6 and discharge heads of Examples 1 and 2 having a configuration as illustrated in FIG. 1 that employ the pressure exertion system of the present invention were prepared and operated in a discharge experiment. The sample discharge heads are denoted by E1 through E8 respectively. In the experiment, the liquid to be discharged was pumped up from a reservoir by means of a pump and fed into each sample discharge head under positive pressure. A pressure sensor is fitted to the discharge head at a position close to the pump relative to the orifice plate and separated from the orifice plate by 10 cm to monitor the pressure of the liquid fed into the discharge head. The discharged liquid was observed by means of a CCD camera, an objective lens and a strobe.

Discharge Head E1 (Prior Art)

A 500 μm-thick ruby-made orifice plate having discharge ports of a diameter of 40 μm was rigidly secured to a head base member by way of a seal member (0-ring). The orifice plate was rigidly held in position from above by means of a plate securing member and screws.

Discharge Head E2 (Prior Art)

A 500 μm-thick ruby-made orifice plate having discharge ports of a diameter of 20 μm was prepared like the discharge head E1.

Discharge Head E3 (Prior Art)

A 25 μm-thick orifice plate made of Ni thin film and having discharge ports of a diameter of 3 μm was prepared by electroforming and bonded to a plate securing member by means of a liquid silicone-based adhesive agent. Then, the plate securing member was rigidly secured to a head base member by means of screws and by way of an O-ring. It was not possible to directly press the O-ring against the orifice plate because the orifice plate was thin.

Discharge Head E4 (Prior Art)

A 25 μm-thick orifice plate made of Ni thin film and having discharge ports of a diameter 1.5 μm was prepared by electroforming like the discharge head E3.

Discharge Head E5 (Prior Art)

A 3 μm-thick orifice plate made of Ni thin film and having discharge ports of a diameter 5 μm was prepared by electroforming and bonded to a plate securing member by means of a thermoplastic solid adhesive agent. Then, the plate securing member was rigidly secured to a head base member by means of screws and by way of an O-ring. It was difficult to handle the orifice plate because the orifice plate was very thin and a solid adhesive agent was employed because it was difficult to uniformly bond the orifice plate to the plate securing member by means of a liquid adhesive agent. As for the discharge ports of the orifice plate, the distance between adjacent discharge ports was 14 μm and the number of discharge ports was 1,000.

Discharge Head E6 (Prior Art)

A 3 μm-thick orifice plate made of Ni thin film and having discharge ports of a diameter of 1.5 μm was prepared by electroforming like the discharge head E5. As for the discharge ports of the orifice plate, the distance between adjacent discharge ports was 7.5 μm and the number of discharge ports was 4,000.

Discharge Head E7 (Example 1)

An orifice plate same as that of the discharge head E1 (ruby-made, 500 μm-thick, having discharge ports of a diameter of 40 μm) was rigidly secured to a head base member as the first orifice plate illustrated in FIG. 1 by way of a seal member (O-ring). Additionally, an orifice plate same as that of the discharge head E5 (Ni-made, prepared by electroforming, 3 μm-thick, having discharge ports of a diameter of 5 μm) was bonded to a second plate securing member in the same manner. The distance separating the first orifice plate and the second orifice plate was 8 mm. It was confirmed that the liquid jets discharged from the first orifice plate were turned into droplets by the time they got to the second orifice plate.

Discharge Head 8 (Example 2)

This discharge head had the same structure as the discharge head E7 except that an orifice plate which is the same as that of the discharge head E6 (Ni-made, prepared by electroforming, 3 μm-thick, having discharge ports of a diameter of 1.5 μm) was used as the second orifice plate.

Since the discharge head E1 and the discharge head E2 employed ruby-made orifice plates that were thick and hard, it was not possible to form discharge ports having a diameter not larger than 20 μm. While it was possible to form very fine discharge ports having a diameter not more than 5 μm through Ni thin film produced by electroforming, the strength of the thin film was remarkably low to make it difficult to rigidly secure the film to a head base member.

Then, refined water was selected as liquid to be discharged and the discharge heads E1 through E8 were operated to discharge droplets. The operations of discharging droplets were observed. As a result of the observation, it was found that all the discharge heads produced liquid jets from the discharge ports and the liquid jets were subsequently split into droplets. The diameter of the droplets was about twice of the diameter of the discharge ports for each discharge head.

As for the discharge head E6, liquid started leaking form the interface of the adhesive agent and the orifice plate to break down the discharge head in 30 seconds after the start of discharging droplets. The discharge head E4 also produced a liquid leakage to a lesser extent so that consequently the pressure in the head fluctuated particularly when the pressure was high and the head was wetted from time to time at and near the discharge ports. The orifice plates of the prior art discharge heads E3 through E6 were deformed to represent a convex profile under water pressure so that some of the discharged liquid jets did not come out perpendicularly relative to the discharge surface of each of the discharge heads.

On the other hand, the orifice plates of the prior art discharge heads E1 and E2 and the discharge heads E7 and E8 of Examples 1 and 2 were not deformed at all. Additionally, all the liquid jets discharged from each of the discharge heads were perpendicular relative to the discharge surface of the head.

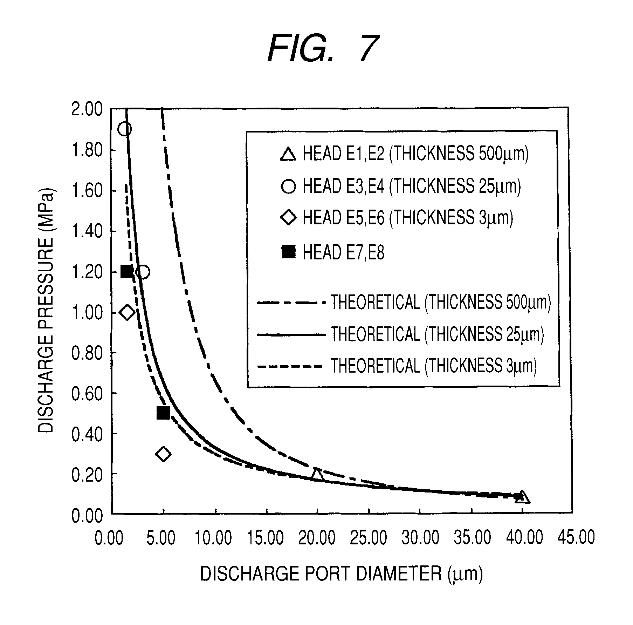

FIG. 7 illustrates the results obtained by observing the discharge pressure of each of the discharge heads when the head started discharging droplets from the discharge ports thereof. The curves of the solid line, the broken line and the chain line in FIG. 7 illustrate the theoretical values of the dependency of discharge pressure on the thickness of ordinary orifice plate that were computationally determined by using formulas (1), (3) and (4) and physical property parameters of refined water and experimentally obtained parameters. The discharge pressure falls as the thickness of the orifice plate decreases and as the nozzle diameter increases. Considering the computationally determined values and the experimentally obtained values, it will be seen that the discharge heads E7 and E8 according to the present invention represent a discharge pressure slightly higher than the prior art discharge heads E5 and E6 having the thinnest orifice plates but lower than the prior art discharge heads E3 and E4. By comparing the present invention and the prior art from the viewpoint of durability of discharge head, it will be seen that the discharge heads E7 and E8 according to the present invention are highly durable relative to the prior art discharge heads E3 through E6.

From the above, it was evidenced that the present invention makes a high discharge durability and a reduced discharge pressure compatible particularly when discharging very fine droplets.

Thus, the present invention can find a broad scope of application including liquid-spraying type medical equipment such as inhalers and nasal drops applicators and equipment for producing a predetermined air flow under the atmospheric pressure such as air conditioners, air cleaners and ventilators, accommodating various modes of use.

The present invention is not limited to the above embodiments and various changes and modifications can be made within the spirit and scope of the present invention. Therefore, to apprise the public of the scope of the present invention, the following claims are made. A discharge head 10 for discharging droplets from discharge ports can withstand high water pressure and is provided with a first orifice plate 3 having first discharge ports 3a and a second orifice plate 6 having second discharge ports. The first orifice plate 3 and the second orifice plate 6 are separated from each other in the liquid discharge direction of the discharge head 10 and disposed opposite to each other. The diameter of the discharge ports of the second orifice plate 6 is smaller than the diameter of the discharge ports 3a of the first orifice plate 3 so that very fine droplets 9 are discharged from the discharge head 10 as the liquid discharged from the discharge ports 3a of the first orifice plate 3 is split by the second discharge ports.

**Claims**

1. A discharge head to be used for a droplet discharging device for discharging liquid from discharge ports by exerting pressure, comprising,
   a first orifice plate having first discharge ports of a first discharge port diameter,
   a second orifice plate arranged at a position separated from the first orifice plate in the liquid discharge direction and having second discharge ports of a second discharge port diameter, the second discharge port diameter being smaller than the first discharge port diameter.

**2.** The discharge head according to claim 1, wherein
the second orifice plate has a plurality of second discharge ports arranged opposite to the first discharge ports.

**3.** The discharge head according to claim 1 or 2, wherein
the liquid discharged from the first discharge ports is turned to droplets before getting to the second orifice plate.

**4.** The discharge head according to any of claims 1 through 3, wherein
the second orifice plate has a thickness smaller than the first orifice plate.

**5.** The discharge head according to any of claims 1 through 4, wherein
the first orifice plate and the second orifice plate satisfy relationship requirements of $T_1 > T_2$, $D_1 > D_2$ and $(T_1 - T_2)$ / $D_2^2 > T_1 / D_1^2$, where $T_1$ is the thickness of the first orifice plate, $D_1$ is the first discharge port diameter, $T_2$ is the thickness of the second orifice plate and $D_2$ is the second discharge port diameter.

**6.** The discharge head according to any of claims 1 through 5, wherein
the distance between adjacent discharge ports of the second orifice plate is not less than the diameter of the second discharge ports and not more than the diameter of the first discharge ports.

**7.** The discharge head according to any of claims 1 through 6, wherein
the diameter of the first discharge ports is not less than 10 $\mu$m and not more than 500 $\mu$m.

**8.** The discharge head according to any of claims 1 through 7, wherein
the distance between the first orifice plate and the second orifice plate is not less than 300 $\mu$m and not more than 50 mm.

**9.** The discharge head according to any of claims 1 through 8, further comprising a liquid collecting section arranged between the first orifice plate and the second orifice plate to collect the liquid that was discharged from the first discharge ports but did not pass through the second discharge ports.

**10.** The discharge head according to any of claims 1 through 9, further comprising an energy application mechanism for applying thermal energy, oscillation energy or charging energy to the second orifice plate.

**11.** The discharge head according to any of claims 1 through 10, wherein
the contact angle of liquid relative to the surface of the second orifice plate is greater than the contact angle of liquid relative to the surface of the first orifice plate.

**12.** A droplet discharging device comprising:

a discharge head according to any of claims 1 through 11;
a pressure exerting mechanism for applying pressure in order to cause the discharge head to discharge liquid; and
a flow path for supplying the liquid pressurized by the pressure exerting mechanism to the discharge head.

**13.** The droplet discharging device according to claim 12, wherein
the liquid ejected from the discharge head is a liquid medicine to be inhaled by a user.

**14.** An inhaler provided with a liquid discharging device according to claim 13.

# FIG. 1

# FIG. 2

# FIG. 3

# FIG. 4

# FIG. 5

# FIG. 6

## FIG. 7

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9501137 A
- WO 9427653 A